(19) Europäisches Patentamt
European Patent Office
Office européen des brevets

(11) **EP 4 318 493 A1**

(12) **EUROPEAN PATENT APPLICATION**
published in accordance with Art. 153(4) EPC

(43) Date of publication:
07.02.2024 Bulletin 2024/06

(21) Application number: 22776137.6

(22) Date of filing: 25.03.2022

(51) International Patent Classification (IPC):
G16H 50/20 (2018.01)      G16H 50/50 (2018.01)
G16H 50/70 (2018.01)      G16B 30/10 (2019.01)
G16B 20/20 (2019.01)      C12Q 1/6869 (2018.01)
C12Q 1/6806 (2018.01)      G06N 20/00 (2019.01)
G06N 3/08 (2023.01)

(52) Cooperative Patent Classification (CPC):
C12Q 1/6806; C12Q 1/6869; G06N 3/08;
G06N 20/00; G16B 20/20; G16B 30/10;
G16H 50/20; G16H 50/50; G16H 50/70

(86) International application number:
PCT/KR2022/004189

(87) International publication number:
WO 2022/203437 (29.09.2022 Gazette 2022/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.03.2021 KR 20210038719

(71) Applicant: GC Genome Corporation
Yongin-si, Gyeonggi-do 16924 (KR)

(72) Inventors:
• CHOI, Jung Kyoon
  Daejeon 34141 (KR)
• KIM, Gyuhee
  Daejeon 34141 (KR)
• CHO, Eun Hae
  Yongin-si Gyeonggi-do 16924 (KR)

(74) Representative: Mewburn Ellis LLP
Aurora Building
Counterslip
Bristol BS1 6BX (GB)

(54) **ARTIFICIAL-INTELLIGENCE-BASED METHOD FOR DETECTING TUMOR-DERIVED MUTATION OF CELL-FREE DNA, AND METHOD FOR EARLY DIAGNOSIS OF CANCER, USING SAME**

(57) The present invention relates to a method for early diagnosis of cancer, using artificial-intelligence-based detection of a tumor-derived mutation of cell-free DNA and, more specifically, to a method for early diagnosis of cancer, using artificial-intelligence-based detection of a tumor-derived mutation of cell-free DNA, the method using a method comprising obtaining sequence information from a biological sample, and then comparing the sequence information with that of a reference genome to detect a mutation, and inputting the detected mutation information into an artificial intelligence model trained to determine the presence of a tumor-derived mutation and analyzing same. A method for detecting a tumor-derived mutation of cell-free DNA, and a method for early diagnosis of cancer, using same, according to the present invention, allow next generation sequencing (NGS) to be used to diagnose cancer early on the basis of artificial intelligence by using both functional and sequence features of cancer, so that high commercial utilization due to high accuracy and sensitivity are provided, and thus the methods of the present invention are useful in early diagnosis of cancer.

EP 4 318 493 A1

FIG. 2

**Description**

[Technical Field]

The present invention relates to an early cancer diagnosis method through detection of tumor-derived mutations in cell-free DNA based on artificial intelligence and more specifically, to an early cancer diagnosis method through detection of tumor-derived mutations in cell-free DNA based on artificial intelligence including obtaining sequence information from a biological sample, comparing the sequence information with a reference genome to detect mutations, and analyzing the detected mutation information by inputting the information into an artificial intelligence model trained to determine the presence of a tumor-derived mutation.

[Background Art]

A major goal of precision oncology is to improve the diagnosis and treatment of cancer. For this purpose, known predictive markers are identified and classification of subtypes of molecules capable of estimating prognosis is induced to select therapies using a variety of genomic and other molecular assays for a tumor material. Also, somatic changes associated with tumor progression are characterized, disrupted pathways are detected and molecular discriminators of metastatic diseases are determined. Although various next-generation sequencing (NGS)-based approaches have been used to characterize the tumor genome in detail, more accurate tumor types may be classified through comprehensive multiparameter analysis. For example, The Cancer Genome Atlas (TCGA) research network has produced comprehensive molecular profiles at the DNA, RNA, protein and epigenetic levels for hundreds of tumors. These multiparametric analyses have advanced our understanding of tumor types, the functional roles of identified new tumor subtypes and molecular variations. Importantly, these efforts have caused identification of novel drug targets, a prerequisite for realizing the promise of precision medicine. However, an approach to tumor materials for molecular profiling is not generally possible, but relies on invasive methods that are not suitable for continuous monitoring of tumor genotypes.

Thus, precision oncology has increasingly focused on liquid biopsies, which are noninvasive, and allow repeated experimentation and easy monitoring of disease. In fact, attempts are being made to use these liquid biopsies for early detection of cancer. The term "liquid biopsy" was first used to describe how the same diagnostic information can be obtained from a blood sample derived from a tissue biopsy sample. In oncology, this term has been used in a broad sense to refer to the assay and sampling of various easily accessible biological fluids such as urine, ascites or pleura as well as blood.

In this case, the analyte of the body fluid peripheral blood contains circulating tumor cells (CTC), circulating cell-free DNA (cfDNA) of cancer patients containing circulating tumor DNA (ctDNA), small RNA, circulating cell-free RNA containing mRNA (cfRNA), circulating extracellular vesicles (EVs) such as exosomes, tumor educated platelets (TEPs), proteins and metabolites. In addition, these analytes have the potential to provide information about the characteristics of primary tumors or metastases commonly obtained by pathologists. In addition to information on genomic mutations and copy number alterations commonly obtained from CTCs or ctDNA, liquid biopsies are used to generate general information on transcripts, protoplasts, proteomes, and metabolomes (Jacob J. Chabon et al., Nature, Vol. 580, pp. 245-25, 2020).

One of the types of liquid biopsy is a method of analyzing small DNA fragments floating in various body fluids including blood with cell free DNA (cfDNA). Research on early diagnosis of cancer using cfDNA is being actively conducted, but there are many issues that need to be improved in studies that accurately analyze single nucleotide variants. cfDNA cancer research using single gene mutations is difficult because most single gene mutations detected through cfDNA are not derived from cancer. The exact detection of tumor-derived mutations is difficult due to very small amounts of single genetic mutations derived from tumors in the blood.

Therefore, many studies, which are being conducted, are limited to well-known single gene mutations that cause cancer, but there are only few mutations that are repeatedly found and the case where the same mutation is found in multiple patients is very rare.

Under such technical background, as a result of extensive and diligent efforts to develop an artificial intelligence-based method for detecting tumor-derived mutations in cell-free DNA and early cancer diagnosis using the method, the present inventors have found that tumor-derived mutations in cell-free DNA can be detected with high sensitivity and accuracy by detecting mutations in the obtained sequence information and inputting the detected mutations into an artificial intelligence model trained to distinguish tumor-derived mutations, and early cancer diagnosis is possible based thereon. Based on this finding, the present invention has been completed.

[Disclosure]

Therefore, it is one object of the present invention to provide an artificial intelligence-based method for detecting a tumor-derived mutation in cell-free DNA.

It is another object of the present invention to provide a method for providing information for early diagnosis of cancer using the detection method.

It is another object of the present invention to provide a method for early diagnosis for cancer using the detection method.

It is another object of the present invention to provide a device and computer-readable storage medium for the method for providing information for early diagnosis of cancer.

It is another object of the present invention to provide a device and computer-readable storage medium for early diagnosis of cancer.

In accordance with one aspect of the present invention, the above and other objects can be accomplished by the provision of an artificial intelligence-based method for detecting a tumor-derived mutation in cell-free DNA, the method including: (a) extracting nucleic acids from a biological sample to obtain sequence information;

(b) aligning the sequence information (reads) with a reference genome database;

(c) detecting a mutation based on the aligned sequence reads; and

(d) inputting the detected mutation information to an artificial intelligence model trained to distinguish a tumor-derived mutation and comparing an output value with a cut-off value to determine whether or not a tumor-derived mutation is present,

wherein the artificial intelligence model in step (d) is trained to distinguish the tumor-derived mutation based on at least one feature selected from the group consisting of a functional feature of cancer, a mutation pattern, and a technical feature of mutation.

In accordance with another aspect of the present invention, provided is a method for providing information for early diagnosis of cancer, the method including: (a) detecting a tumor-derived mutation in cell-free DNA by the method described above; and (b) determining that cancer or microscopic residual cancer is present when the tumor-derived mutation is detected.

In accordance with another aspect of the present invention, provided is an artificial intelligence-based device for providing information for early diagnosis of cancer, the device including: a decoder configured to extract nucleic acids from a biological sample and decode sequence information; an aligner configured to align the decoded sequence with a reference genome database; a mutation detector configured to detect a mutation based on the aligned sequence information; a tumor-derived mutation detector configured to input the detected mutation into an artificial intelligence model trained to distinguish a tumor-derived mutation and determine whether or not a tumor-derived mutation is present; and a cancer diagnostic unit configured to determine that cancer or microscopic residual cancer is present when the tumor-derived mutation is detected.

In accordance with another aspect of the present invention, provided is a computer-readable storage medium including an instruction configured to be executed by a processor for providing information for early diagnosis of cancer, through the following steps including: (a) extracting nucleic acids from a biological sample to obtain sequence information; (b) aligning the sequence information (reads) with a reference genome database; (c) detecting a mutation based on the aligned sequence reads; (d) inputting the detected mutation information to an artificial intelligence model trained to distinguish a tumor-derived mutation and comparing an output value with a cut-off value to determine whether or not a tumor-derived mutation is present; and (e) determining that cancer or microscopic residual cancer is present when the tumor-derived mutation is detected, wherein the artificial intelligence model in step (d) is trained to distinguish the tumor-derived mutation based on at least one feature selected from the group consisting of a functional feature of cancer, a mutation pattern, and a technical feature of mutation.

In accordance with another aspect of the present invention, provided is a method for early diagnosis of cancer, the method including: (a) detecting a tumor-derived mutation in cell-free DNA by the method described above; and (b) determining that cancer or microscopic residual cancer is present when the tumor-derived mutation is detected.

In accordance with another aspect of the present invention, provided is an artificial intelligence-based device for early diagnosis of cancer, the device including: a decoder configured to extract nucleic acids from a biological sample and decode sequence information; an aligner configured to align the decoded sequence with a reference genome database; a mutation detector configured to detect a mutation based on the aligned sequence information; a tumor-derived mutation detector configured to input the detected mutation into an artificial intelligence model trained to distinguish a tumor-derived mutation and determine whether or not a tumor-derived mutation is present; and a cancer diagnostic unit configured to determine that cancer or microscopic residual cancer is present when the tumor-derived mutation is detected.

In accordance with another aspect of the present invention, provided is a computer-readable storage medium including an instruction configured to be executed by a processor for early diagnosis of cancer, through the following steps including: (a) extracting nucleic acids from a biological sample to obtain sequence information; (b) aligning the sequence information (reads) with a reference genome database; (c) detecting a mutation based on the aligned sequence reads; (d) inputting the detected mutation information to an artificial intelligence model trained to distinguish a tumor-derived mutation and comparing an output value with a cut-off value to determine whether or not a tumor-derived mutation is present; and (e) determining that cancer or microscopic residual cancer is present when the tumor-derived mutation is detected, wherein the artificial intelligence model in step (d) is trained to distinguish the tumor-derived mutation based on at least one feature selected from the group consisting of a functional feature of cancer, a mutation pattern, and a technical feature of mutation.

[Description of Drawings]

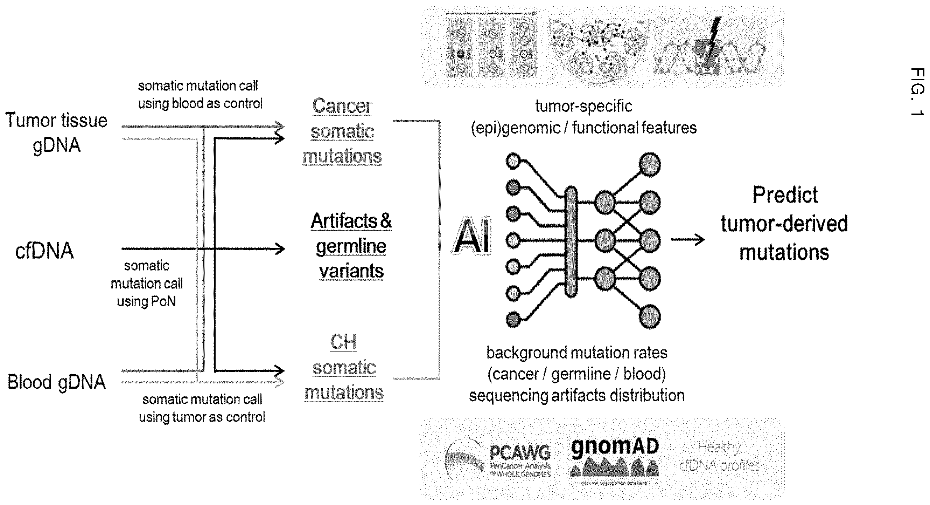

FIG. 1 is an overall flowchart illustrating a method for early diagnosis of cancer based on artificial intelligence according to the present invention.

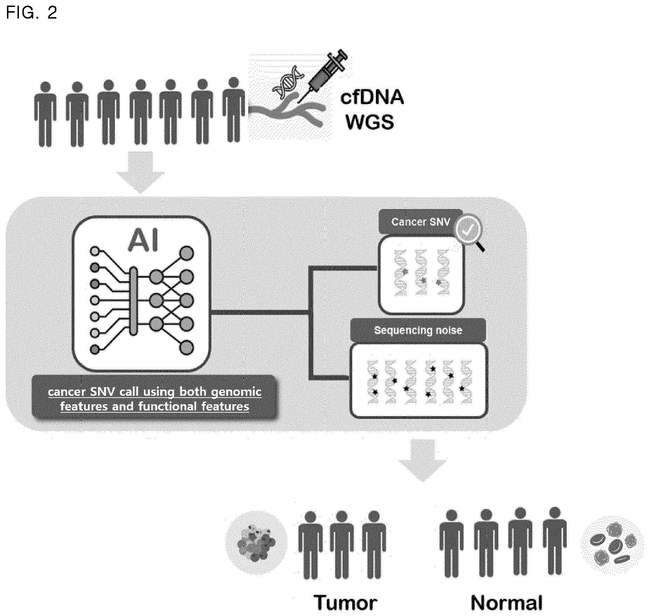

FIG. 2 is an overall flowchart illustrating a method for early diagnosis of cancer based on artificial intelligence according to the present invention.

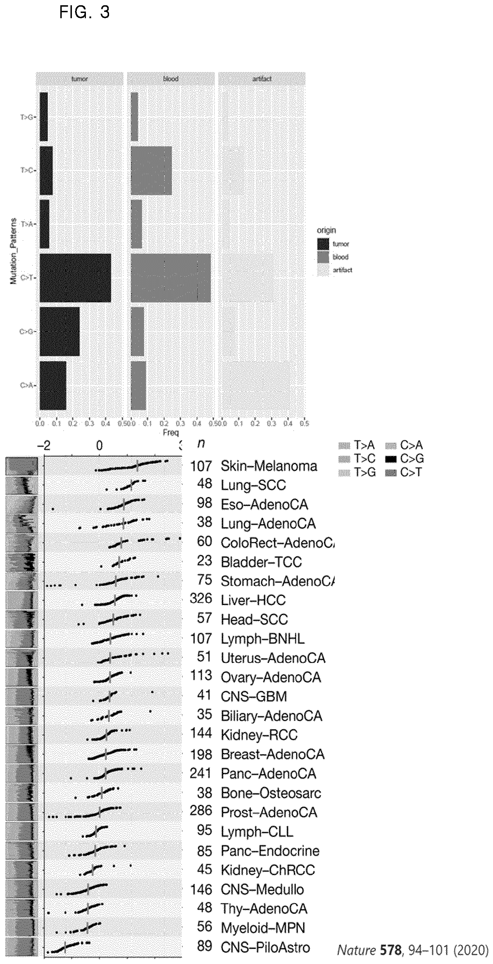

FIG. 3 show the result of analysis of the characteristics depending on each origin of the single gene mutation of cell-free DNA detected according to an embodiment of the present invention, wherein the top panel represents a mutational signature by origin of a single genetic mutation in cell-free DNA of a breast cancer patient analyzed according to an embodiment and the bottom panel represents a mutational signature in cancer tissue of the patient depending on the type of cancer conducted in a large-scale cancer genome project called "Pan-cancer Analysis of Whole Genomes (PCAWG)", wherein the mutational signature is based on the concept that there is a pattern specific for the type of single gene mutation that occurs in a specific cancer type.

FIG. 4 shows the result of determination as to the distribution of breast cancer biological features depending on the origin of cfDNA in breast cancer patients, wherein (A) show the result of determination as to the replication score, H3K9me3 and gene expression level, and (B) represents a single gene mutation accumulation pattern (regional mutation density, RMD).

FIG. 5 shows the result of determination as to the performance of a breast cancer-derived single gene mutation detection training model constructed according to an embodiment of the present invention, wherein (A) is an ROC curve showing the performance of a classification model using sensitivity and specificity, and (B) is a PR curve showing the performance of the classification model using precision and recall.

FIG. 6 shows the result of evaluation as to the importance of respective features used in the training model constructed according to an embodiment of the present invention.

FIG. 7 shows the result of comparison between a mutational signature predicted using the training model constructed according to an embodiment of the present invention and an actual result.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

Terms such as first, second, A, B, and the like may be used to describe various elements, but these elements are not limited by these terms and are merely used to distinguish one element from another. For example, without departing from the scope of the technology described below, a first element may be referred to as a second element and in a similar way, the second element may be referred to as a first element. "And/or" includes any combination of a plurality of related recited items or any one of a plurality of related recited items.

Singular forms are intended to include plural forms as well, unless the context clearly indicates otherwise. It will be further understood that the terms "comprises" and/or "comprising", when used in this specification, specify the presence of features, numbers, steps, actions, components, parts, or combinations thereof, but do not preclude the presence or addition of one or more other features, numbers, steps, actions, components, parts, or combinations thereof.

Prior to the detailed description of the drawings, it is to be clarified that the classification of components in the present specification is merely made depending on the main function of each component. That is, two or more components described below may be combined into one component or one component may be divided into two or more depending on each more detailed function. In addition, each component to be described below may further perform some or all of the functions of other components in addition to its main function, and some of the main functions of each component may be performed exclusively by other components.

In addition, in implementing a method or operation method, respective steps constituting the method may occur in a different order from a specific order unless the specific order is clearly described in context. That is, the steps may be performed in the specific order, substantially simultaneously, or in reverse order to that specified.

The present invention is intended to determine whether or not tumor-derived mutations in cell-free DNA can be detected with high sensitivity and accuracy by aligning sequencing data obtained from a sample with a reference genome database, detecting mutations in the aligned nucleic acid fragments, and inputting the detected mutation information into an artificial intelligence model trained to distinguish tumor-derived mutations.

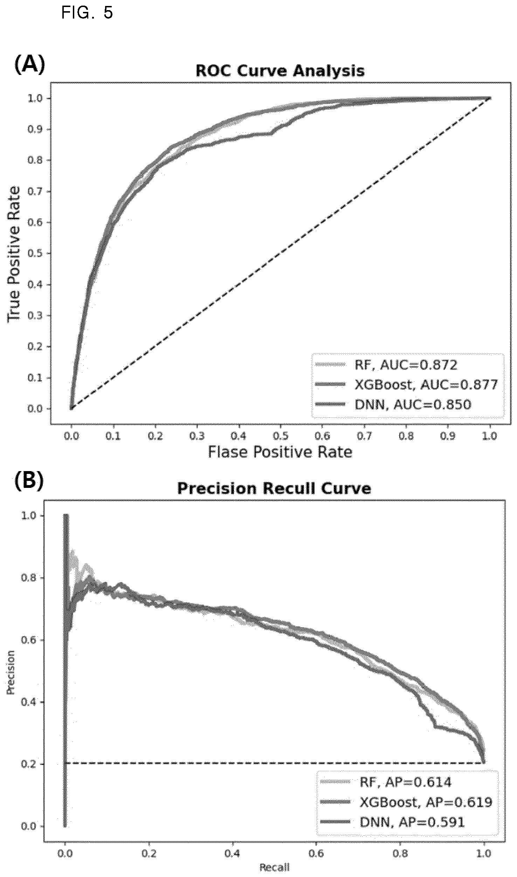

That is, in one embodiment of the present invention, a training model capable of detecting tumor-derived mutations was constructed with 48 features including functional features and sequencing quality features of cancer, the performance was tested using cfDNA, tumor, and WBC liquid biopsies of 38 breast cancer patients, and the result showed that the performance was excellent (FIG. 5).

As used herein, the term "read" refers to a single nucleic acid fragment, sequence information of which is analyzed using various methods known in the art. Therefore, the terms "sequence information" and "read" have the same meaning in that both are sequence information obtained through a sequencing process.

As used herein, the term "tumor-derived mutation" refers to a mutation that occurs in cancer cells.

In one aspect,
the present invention is directed to an artificial intelligence-based method for detecting a tumor-derived mutation in cell-free DNA, the method including:

(a) extracting nucleic acids from a biological sample to obtain sequence information;

(b) aligning the sequence information (reads) with a reference genome database;

(c) detecting a mutation based on the aligned sequence reads; and

(d) inputting the detected mutation information to an artificial intelligence model trained to distinguish a tumor-derived mutation and comparing an output value with a cut-off value to determine whether or not a tumor-derived mutation is present,
wherein the artificial intelligence model in step (d) is trained to distinguish the tumor-derived mutation based on at least one feature selected from the group consisting of a functional feature of cancer, a mutation pattern, and a technical feature of mutation.

In the present invention, step (a) to obtain sequence information includes:

(a-i) obtaining nucleic acids from a biological sample;
(a-ii) removing proteins, fats, and other residues from the obtained nucleic acids using a salting-out method, a column chromatography method, or a bead method to obtain purified nucleic acids;
(a-iii) producing a single-end sequencing or paired-end sequencing library for the purified nucleic acids or nucleic acids randomly fragmented by enzymatic digestion, pulverization, or hydroshearing;
(a-iv) reacting the produced library with a next-generation sequencer; and
(a-v) obtaining sequence information (reads) of the nucleic acids in the next-generation sequencer.

In the present invention, the step (a) to obtain sequence information may include obtaining the isolated cell-free DNA through whole genome sequencing at a depth of 1 million to 100 million reads.

In the present invention, the biological sample refers to any substance, biological fluid, tissue or cell obtained from or derived from a subject, and examples thereof include, but are not limited to, whole blood, leukocytes, peripheral blood mononuclear cells, leukocyte buffy coat, blood including plasma and serum, sputum, tears, mucus, nasal washes, nasal aspirates, breath, urine, semen, saliva, peritoneal washings, pelvic fluids, cystic fluids, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extracts, semen, hair, saliva, urine, oral cells, placenta cells, cerebrospinal fluid, and mixtures thereof.

As used herein, the term "reference population" refers to a reference group that is used for comparison like a reference genome database and refers to a population of subjects who do not currently have a specific disease or condition. In the present invention, the reference nucleotide sequence in the reference genome database of the reference population may be a reference chromosome registered with public health institutions such as the NCBI.

In the present invention, the nucleic acid in step (a) may be cell-free DNA, more preferably circulating tumor DNA, but is not limited thereto.

In the present invention, the next-generation sequencer may be used for any sequencing method known in the art. Sequencing of nucleic acids isolated using the selection method is typically performed using next-generation sequencing (NGS). Next-generation sequencing includes any sequencing method that determines the nucleotide sequence either of each nucleic acid molecule or of a proxy cloned from each nucleic acid molecule so as to be highly similar thereto (e.g., 105 or more molecules are sequenced simultaneously). In one embodiment, the relative abundance of nucleic acid species in the library can be estimated by counting the relative number of occurrences of the sequence homologous thereto in data produced by sequencing experimentation. Next-generation sequencing is known in the art, and is described, for example, in Metzker, M. (2010), Nature Biotechnology Reviews 11:31-46, which is incorporated herein by reference.

In one embodiment, next-generation sequencing is performed to determine the nucleotide sequence of each nucleic acid molecule (using, for example, a HelioScope Gene-Sequencing system from Helicos Biosciences or a PacBio RS system from Pacific Biosciences) . In other embodiments, massive parallel short-read sequencing, which produces more bases of the sequence per sequencing unit than other sequencing methods, for example, other sequencing methods that produce fewer but longer reads, determines the nucleotide sequence of a proxy cloned from each nucleic acid molecule (using, for example, a Solexa sequencer from Illumina Inc., located in San Diego, CA; 454 Life Sciences (Branford, Connecticut) and Ion Torrent). Other methods or devices for next-generation sequencing may be provided by 454 Life Sciences (Branford, Connecticut), Applied Biosystems (Foster City, CA; SOLiD Sequencer), Helicos Biosciences Corporation (Cambridge, MA) and emulsion and microfluidic sequencing nanodrops (e.g., GnuBIO Drops), but are not limited thereto.

Platforms for next-generation sequencing include, but are not limited to, the FLX System genome sequencer (GS) from Roche/454, the Illumina/Solexa genome analyzer (GA), the Support Oligonucleotide Ligation Detection (SOLiD) system from Life/APG, the G.007 system from Polonator, the HelioScope gene-sequencing system from Helicos Biosciences, and the PacBio RS system from Pacific Biosciences.

In the present invention, the alignment of step (b) may be performed using the BWA algorithm and the Hg19 sequence, but is not limited thereto.

In the present invention, the BWA algorithm may include BWA-ALN, BWA-SW or Bowtie2, but is not limited thereto.

In the present invention, the method may further include selecting reads having a mapping quality score of the aligned nucleic acid fragments equal to or greater than a cut-off value prior to step (c), wherein any value capable of confirming the quality of the aligned nucleic acid fragments may be used as the cut-off value without limitation and the cut-off value is preferably 50 to 70, more preferably 60, but is not limited thereto.

In the present invention, the step of detecting the mutation in step (c) may include:

(c-i) selecting a nucleotide sequence different from the reference genome in the aligned reads; and
(c-ii) storing the selected nucleotide sequence information.

In the present invention, step (c-i) may use any method known to those skilled in the art capable of detecting mutations, preferably Mutect2, LoFreq, Delly2, and the like, but is not limited thereto.

In the present invention, in step (c-ii), the sequence information may be stored in a specific file format or as the mutation information detected in step (c-i).

In the present invention, the functional feature of cancer may be used without limitation as long as it is a genomic, epigenomic, or transcriptome feature that affects the occurrence of single genetic mutations for each cancer type, and preferably include one or more selected from the group consisting of a single genetic mutation accumulation pattern (regional mutation density, RMD), replication timing, H3K4Me1, H3K4Me3, H3K9Me3, H3K27Me3, H3K36Me3, Dnase I hypersensitive site (DHS), an amount of protein binding site (footprint) gene expression in DHS, a cancer positive selection score and a cancer negative selection score, but is not limited thereto.

In the present invention, the single genetic mutation accumulation pattern (regional mutation density, RMD) is used as a similar meaning to the background mutation rate and means that the regional mutation density (RMD) means a mutation frequency calculated in a certain section of the whole genome.

In the present invention, the single gene mutation accumulation pattern (regional mutation density, RMD) for each type of cancer is a quantitative value indicating whether the cancer has a high or low mutation rate. The cancer single gene mutation is not evenly distributed in the human genome. The amount of single gene mutations accumulated varies depending on the section of the whole genome and the accumulation pattern is also very different for each cancer type. In addition, the epigenetic feature (histone modification, replication timing) is the main cause of the single gene mutation accumulation pattern for each cancer type, and the single gene mutation accumulation pattern implies the epigenetic feature of the cancer type.

The single gene mutation accumulation pattern may be a beneficial indicator for detecting tumor-derived mutations because it is different for each genome region and cancer type. The single gene mutation accumulation pattern indicates whether or not the detected mutation is located in a region with a high probability of occurrence in the cancer. The mutation detected in regions with a high probability of mutation in the cancer are likely to be an actual tumor-derived mutation, not a cfDNA artifact. In addition, the single gene mutation accumulation pattern also includes epigenomic features. Epigenomic features may also be considered for the detection of tumor-derived mutations.

In addition, haematopoiesis mutation accumulation patterns are used to determine regions in blood cells where mutations are easily generated, normal cell-free mutation accumulation patterns are used to determine areas where cfDNA artifacts are easily discovered, and normal germline mutation accumulation patterns are used to determine areas where germline mutations are likely to occur.

WGS of a sufficient number of samples from a large cohort is required to calculate the single gene accumulation pattern. The single gene mutation accumulation pattern is calculated by summing all mutations found in the sample.

The single gene accumulation pattern (regional mutation density, RMD) is calculated as the mutation frequency in a certain section, for example, 10 kb or 1 Mb, divided from the entire genome, and normalization is performed by dividing the amount of mutation in each section by the number of mutations found in the entire genome.

It is important to set an appropriate section because when the section divided from the whole genome is short (e.g., 1 kb), it may be difficult to detect the pattern due to excessive small area, and when the section is long (e.g., 10 Mb), local patterns may be aggregated.

In the present invention, any mutation may be used without limitation as the mutation pattern as long as it is a mutation that causes functional abnormality of genes due to modification of a normal base with another base, and the mutation pattern preferably includes at least one selected from the group consisting of C -> A, C -> G, C -> T, T -> A, T -> C and T -> G, but is not limited thereto.

In the present invention, C->A means a detected mutation in which a normal base C is mutated to a mutant base A, C->G means a detected mutation in which a normal base C is mutated to a mutant base G, and the remaining has the same meaning.

In the present invention, the technical feature of mutation may be used without limitation as long as it is a feature of sequence information extracted from sequence information (reads) aligned with the single genetic mutation site, and preferably includes, but is not limited to, at least one selected from the group consisting of an average read depth, an average mapping quality, an average base quality, an average number of mismatches, an average of reference allele positions, an average of base quality sums of mismatches, the number or position of bases having a Phred quality of 2 at a 3' end, an average clipped read length, an average of positions from a read 3' end, a ratio of plus strand reads, and a DNA fragment length of a reference allele of the mutation region;

an average read depth, an average mapping quality, an average base quality, an average number of mismatches, an average of reference allele positions, an average of base quality sums of mismatches, the number or position of bases having a Phred quality of 2 at a 3' end, an average clipped read length, an average of positions from the read 3' end, a ratio of plus strand reads, a DNA fragment length and a DNA fragment ratio of a variant allele of the mutation region; and

MUT.notBoth (defined as the number of DNA fragments that do not overlap at mutation positions in forward and reverse reads + the number of DNA fragments that overlap at mutation positions in forward and reverse reads, but have different mutations).

In the present invention, the feature of step (d) may include the features described in Table 1 below.

[Table 1]

| Feature List | | | | | |
|---|---|---|---|---|---|
| Feature name | type | specific_ty pe | Too l | Sample | Description |
| pcawg_ tumor_R MD | biologi cal | tissue-specific | . | PCAWG cohort | Cancer patient-derived tissue specific background mutation rate. Mutation frequency calculated in each section of genome |
| pcawg_ blood_R MD | biologi cal | blood | . | PCAWG cohort | Background mutation rate of haematopoiesi s (blood) mutation |

(continued)

| Feature List | | | | | |
|---|---|---|---|---|---|
| Feature name | type | specific_ ty pe | Too l | Sample | Description |
| normal_ cfDNA_ RMD | biologi cal | normal | . | Normal subjec t cfDNA | Background mutation rate of cell-free DNA of normal subject |
| gnomad_ RMD | biologi cal | germline | . | Gnomad cohort | Background mutation rate of germline mutation of normal subject |
| repli_score | biologi cal | tissue- specific | . | Cell line of cancer | Relative replication timing for each genomic region |
| H3K4me1 | biologi cal | tissue- specific | . | Cell line of cancer | Signal for each genomic region of H3K4me1 histone modification |
| H3K4me3 | biologi cal | tissue- specific | . | Cell line of cancer | Signal for each genomic region of H3K4me3 histone modification |
| H3K9me3 | biologi cal | tissue- specific | . | Cell line of cancer | Signal for each genomic region of H3K9me3 histone modification |
| H3K27me3 | biologi cal | tissue- specific | . | Cell line of cancer | Signal for each genomic region of H3K27me3 histone modification |
| H3K36me3 | biologi cal | tissue- specific | . | Cell line of cancer | Signal for each genomic region of H3K36me3 histone modification |
| DHS | biologi cal | tissue- specific | . | Cell line of cancer | Dnase 1 hypersensitive site (DHS) of certain cancer type |
| DHS_all | biologi cal | pan- cancer | . | Cell line of all cancer s | Dnase 1 hypersensitiv e site (DHS) of all cancer types |
| tcga_expressi on | biologi cal | tissue- specific | . | TCGA cohort | Gene expression levels in specific cancer type |
| cancer_pos | biologi cal | tissue- specific | . | 10.101 6/j.ce 11.201 7.09.0 42, 10.103 8/ng. 3 987 | Score for genes more prone to mutation due to positive selection as cancer progresses |
| cancer_neg | biologi cal | tissue- specific | . | 10.101 6/j.ce 11.201 7.09.0 42 | Score for genes less prone to mutation due to negative selection as cancer progresses |
| footprint | biologi cal | pan- cancer | . | 10.103 8/s415 86-020-2819-2 | Protein (e.g. TF) binding site in DHS of all cancer types |
| C2A | mutatio n pattern | . | . | . | Whether or not the mutation is a C->A mutation |
| C2G | mutatio n pattern | . | . | . | Whether or not the mutation is a C->G mutation |
| C2T | mutatio n pattern | . | . | . | Whether or not the mutation is a C->T mutation |
| T2A | mutatio n pattern | . | . | . | Whether or not the mutation is a T->A mutation |

(continued)

| Feature List | | | | | |
|---|---|---|---|---|---|
| Feature name | type | specific_ ty pe | Too l | Sample | Description |
| T2C | mutatio n pattern | . | . | . | Whether or not the mutation is a T->C mutation |
| T2G | mutatio n pattern | . | . | . | Whether or not the mutation is a T->G mutation |
| non_ref_alt_m eanCount | technic al | | bam cou nt | . | Average read depth of bases excluding reference or variant alleles of mutation region |
| ref_avg_ mappi ng_ quality | technic al | . | bam cou nt | . | Average mapping quality of reference allele of the corresponding mutation region |
| ref_avg_ baseq uality | technic al | . | bam cou nt | . | Average base quality of reference allele of the corresponding mutation region |
| ref_avg_pos_ a s_fraction | technic al | . | bam cou nt | . | Average at reference allele positions in reads including reference allele of the corresponding mutation region |
| ref_avg_num_ m ismatches_ as_ fraction | technic al | . | bam cou nt | . | Average number of mismatches in reads including reference allele of the corresponding mutation region |
| ref_avg_sum_ m ismatch_ quali ties | technic al | . | bam cou nt | . | Average of base quality sums of mismatches present in reads including reference allele of the corresponding mutation region |
| ref_num_ q2_co ntaining_read s | technic al | . | bam cou nt | . | The number of bases having a Phred quality of 2 at 3' end of reads including reference allele of the corresponding mutation region |
| ref_avg_dista nce_to_ q2_sta rt_in_ q2_read s | technic al | . | bam cou nt | . | The position of bases having a Phred quality of 2 at 3' end of reads including reference allele of the corresponding mutation region |
| ref_avg_clipp ed_length | technic al | . | bam cou nt | . | Average clipped read length of reads including reference allele of the corresponding mutation region |
| ref_avg_dista nce_to_effect ive_3p_end | technic al | . | bam cou nt | . | Average of positions from read 3' end of reference allele of the corresponding mutation region |
| ref_plus_stra nd_ratio | technic al | . | bam cou nt | . | Ratio of plus strand read in reads including reference allele of the corresponding mutation region |
| alt_avg_ mappi ng_ quality | technic al | . | bam cou nt | . | Average mapping quality of variant allele of the corresponding mutation region |

(continued)

| Feature List | | | | | |
|---|---|---|---|---|---|
| Feature name | type | specific_ty pe | Too l | Sample | Description |
| alt_avg_ baseq uality | technic al | . | bam cou nt | . | Average base quality of variant allele of the corresponding mutation region |
| alt_avg_pos_ a s_fraction | technic al | . | bam cou nt | . | Average of variant allele positions in reads including variant allele of the corresponding mutation region |
| alt_avg_num_ m ismatches_ as_ fraction | technic al | . | bam cou nt | . | Average number of mismatches in reads including variant allele of the corresponding mutation region |
| alt_avg_sum_ m ismatch_ quali ties | technic al | . | bam cou nt | . | Average of base quality sums of mismatches in reads including variant allele of the corresponding mutation region |
| alt_num_ q2_co ntaining_read s | technic al | . | bam cou nt | . | The number of bases having a Phred quality of 2 at 3' end of reads including variant allele of the corresponding mutation region |
| alt_avg_dista nce_to_ q2_sta rt_in_ q2_read s | technic al | . | bam cou nt | . | The position of bases having a Phred quality of 2 at 3' end of reads including variant allele of the corresponding mutation region |
| alt_avg_clipp ed_length | technic al | . | bam cou nt | . | Average clipped read length of reads including variant allele of the corresponding mutation region |
| alt_avg_dista nce_to_effect ive_3p_end | technic al | . | bam cou nt | . | Average of positions from read 3' end of variant allele of the corresponding mutation region |
| alt_plus_stra nd_ratio | technic al | . | bam cou nt | . | Ratio of plus strand read in reads including variant allele of the corresponding mutation region |
| frag_length | technic al | .. | pyt hon | . | DNA fragment length of the corresponding mutation region |
| ref_frag_leng th | technic al | . | pyt hon | . | DNA fragment length including reference allele of the corresponding mutation region |
| mut_frag_leng th | technic al | . | pyt hon | . | DNA fragment length including variant allele of the corresponding mutation region |
| mut_frag_rati o | technic al | . | pyt hon | . | (DNA fragment length including variant allele of the corresponding mutation region) / (DNA fragment length of the corresponding mutation region) |
| MUT.notBoth | technic al | . | pyt hon | . | the number of DNA fragments that do not overlap at the mutation position in forward and reverse reads + the number of DNA fragments that overlap at the mutation position in forward and reverse reads, but have different mutations. |

In the present invention, any model may be used as the artificial model in step (d) without limitation as long as it is a model trained to distinguish whether a tumor-derived mutation is correct or not and is preferably selected from the group consisting of random forest, XGboost, and deep neural network, but is not limited thereto.

In the present invention, the cut-off value in step (d) can be used without limitation as long as it is a value used to distinguish whether or not the detected mutation is derived from a tumor, and may be preferably 0.5, but is not limited thereto. When the cut-off value is 0.5, a case with an output of 0.5 or more is determined to be derived from a tumor.

In the present invention, the artificial intelligence model is trained to adjust an output value to about 1 if there is a tumor-derived mutation and to adjust an output value to about 0 if there is no tumor-derived mutation. Therefore, the artificial intelligence model is trained based on a cut-off value of 0.5. In other words, the artificial intelligence model is trained such that, if the output value is 0.5 or more, it is determined that there is cancer, and if the output value is less than 0.5, it is determined that there is no cancer.

Here, it will be apparent to those skilled in the art that the cut-off value of 0.5 may be arbitrarily changed. For example, in an attempt to reduce false positives, the cut-off value may be set to be higher than 0.5 as a stricter criterion for determining whether or not there is cancer, and in an attempt to reduce false negatives, the cut-off value may be set to be lower than 0.5 as a weaker criterion for determining that there is cancer.

In the present invention, to evaluate training and performance of the artificial intelligence model, 38 breast cancer patients were divided into the training set (30 persons) and the test set (8 persons) at a ratio of 8:2. 2418 cell-free tumor-derived mutations and 8749 artifacts from 30 breast cancer patients were used for the training set, and 1159 cell-free tumor-derived mutations and 2441 artifacts from 8 breast cancer patients were used for the test set. In addition, for DNN model training and testing, the training set (30 people) was divided into the training set and the validation set at a ratio of 3:1.

In the present invention, when the artificial intelligence model is a random forest, the loss function is represented by Equation 1 or 2 below.

Equation 1:

$$\theta_j^* = \arg\max_{\theta_j \in \mathcal{T}_j} I_j,$$

$$I_j = I(S_j, S_j^{\mathrm{L}}, S_j^{\mathrm{R}}, \theta_j) = H(S_j) - \sum_{i \in \{\mathrm{L,R}\}} \frac{|S_j^i|}{|S_j|} H(S_j^i).$$

If $\tau$ is defined as a set that includes all possible values of the parameter $\theta$ of the node split function, a subset $\tau i$ satisfying $\tau i \subset \tau$ is created at the training stage of the $j^{th}$ node. The optimal parameter $\theta_j^*$ is calculated as a value that maximizes the target function (loss function) defined as information gain in Ti.

Equation 2: $\quad I = H(S) - \sum_{i \in \{\mathrm{L,R}\}} \frac{|S^i|}{|S|} H(S^i),$

wherein I represents an amount of the obtained information, S represents a data set reaching one node, $S^i$ represents a data set entering i E{L,R}, left or right child nodes of the corresponding node, and | • | and H(S) represent the number of data pertaining to the data set and Shannon entropy, respectively.

In the present invention, when the artificial intelligence model is XGBoost, the loss function is represented by the following Equation 3.

Equation 3: $\quad \mathcal{L}(\phi) = \sum_i l(\hat{y}_i, y_i) + \sum_k \Omega(f_k)$

wherein I represents a differentiable convex loss function that computes the difference between the predicted value $\hat{y}$ and the actual value y, $\Omega$ gives a penalty to the complexity of the model, and $f_k$ represents an independent tree structure.

In the present invention, when the artificial intelligence model is a deep neural network, the loss function may be represented by Equation 4 below.

Equation 4:

$$BCE = -\frac{1}{N}\sum_{i=0}^{N} y_i \cdot log(\hat{y}_i) + (1 - y_i) \cdot log(1 - \hat{y}_i)$$

wherein the loss function is binary cross entropy, N is the total number of samples, $\hat{y}_i$ is the probability that the model predicts that the $i^{th}$ input value is close to class 1, and $y^i$ is the actual class of the $i^{th}$ input value.

In the present invention, when the artificial intelligence model is a DNN, the training includes the following steps:

i) classifying the detected mutation data into training, validation, and test data,
wherein the training data is used to train the artificial intelligence model, the validation data is used to validate hyper-parameter tuning, and the test data is used for the test after optimal model production; and
ii) constructing an optimal artificial intelligence model through hyper-parameter tuning and training; and
iii) comparing the performance of multiple models obtained through hyper-parameter tuning using the validation data and determining the model having the best validation data as the optimal model.

In the present invention, hyper-parameter tuning is a process of optimizing the values of various parameters (the number of convolution layers, the number of dense layers, the number of convolution filters, etc.) constituting the artificial intelligence model. Hyper-parameter tuning is performed using Bayesian optimization and grid search methods.

In the present invention, the internal parameters (weights) of the artificial intelligence model are optimized using predetermined hyper-parameters, and it is determined that the model is over-fit when validation loss starts to increase compared to training loss and then training is stopped.

In the present invention, any value resulting from analysis of the input vectorized data by the artificial intelligence model in step (e) may be used without limitation, as long as it is a specific score or real number, and the value is preferably a real number, but is not limited thereto.

In the present invention, when the artificial intelligence model is a DNN, the real number means a value expressed as a probability value by adjusting the output of the artificial intelligence model to a scale of 0 to 1 using applying the sigmoid function or SoftMax function for the last layer.

In another aspect,
the present invention is directed to a method for providing information for early diagnosis of cancer, the method including:

(a) detecting a tumor-derived mutation in cell-free DNA by the method described above; and

(b) determining that cancer or microscopic residual cancer is present when the tumor-derived mutation is detected.

In another aspect,

the present invention is directed to an artificial intelligence-based device for providing information for early diagnosis of cancer, the device including: a decoder configured to extract nucleic acids from a biological sample and decode sequence information;

an aligner configured to align the decoded sequence with a reference genome database;

a mutation detector configured to detect a mutation based on the aligned sequence information;

a tumor-derived mutation detector configured to input the detected mutation into an artificial intelligence model trained to distinguish a tumor-derived mutation and determine whether or not a tumor-derived mutation is present; and

a cancer diagnostic unit configured to determine that cancer or microscopic residual cancer is present when the tumor-derived mutation is detected.

In the present invention, the decoder may include a nucleic acid injector configured to inject the nucleic acid extracted from an independent device, and a sequence information analyzer configured to analyze the sequence information of the injected nucleic acid, preferably an NGS analyzer, but is not limited thereto.

In the present invention, the decoder may receive and decode sequence information data generated in the independent device.

In another aspect,
the present invention is directed to a computer-readable storage medium including an instruction configured to be executed by a processor for providing information for early diagnosis of cancer, through the following steps including:

(a) extracting nucleic acids from a biological sample to obtain sequence information;

(b) aligning the sequence information (reads) with a reference genome database;

(c) detecting a mutation based on the aligned sequence reads;

(d) inputting the detected mutation information to an artificial intelligence model trained to distinguish a tumor-derived mutation and comparing an output value with a cut-off value to determine whether or not a tumor-derived mutation is present; and

(e) determining that cancer or microscopic residual cancer is present when the tumor-derived mutation is detected, wherein the artificial intelligence model in step (d) is trained to distinguish the tumor-derived mutation based on at least one feature selected from the group consisting of a functional feature of cancer, a mutation pattern, and a technical feature of mutation.

In another aspect, the method according to the present disclosure may be implemented using a computer. In one embodiment, the computer includes one or more processors coupled to a chipset. In addition, a memory, a storage device, a keyboard, a graphics adapter, a pointing device, a network adapter and the like are connected to the chipset. In one embodiment, the performance of the chipset is acquired by a memory controller hub and an I/O controller hub. In another embodiment, the memory may be directly coupled to a processor instead of the chipset. The storage device is any device capable of maintaining data, including a hard drive, compact disc read-only memory (CD-ROM), DVD, or other memory devices. The memory relates to data and instructions used by the processor. The pointing device may be a mouse, track ball or other type of pointing device, and is used in combination with a keyboard to transmit input data to a computer system. The graphics adapter presents images and other information on a display. The network adapter is connected to the computer system through a local area network or a long distance communication network. However, the computer used herein is not limited to the above configuration, may not have some configurations, may further include additional configurations, and may also be part of a storage area network (SAN), and the computer of the present invention may be configured to be suitable for the execution of modules in the program for the implementation of the method according to the present invention.

The module used herein may mean a functional and structural combination of hardware to implement the technical idea according to the present invention and software to drive the hardware. For example, it is apparent to those skilled in the art that the module may mean a logical unit of predetermined code and a hardware resource to execute the predetermined code, and does not necessarily mean physically connected code or one type of hardware.

In another aspect, the present invention is directed to a method for early diagnosis of cancer, the method including: (a) detecting a tumor-derived mutation in cell-free DNA by the method described above; and (b) determining that cancer or microscopic residual cancer is present when the tumor-derived mutation is detected.

In another aspect, the present invention is directed to a method of treating a cancer patient, including (a) detecting tumor-derived mutations in cell-free DNA by the method described above; (b) determining that there is cancer or microscopic residual cancer when a tumor-derived mutation is detected; and (c) treating a patient determined to have cancer or microscopic residual cancer.

In the present invention, the cancer therapy may be used without limitation as long as it can treat cancer or microscopic residual cancer and is preferably performed with one or more selected from the group consisting of surgery, adjuvant chemotherapy, neoadjuvant chemotherapy, radiation therapy, hormone therapy, cytotoxic therapy, immunotherapy, adaptive T cell therapy, targeted therapy, and combinations thereof, is more preferably performed by administering a cancer therapeutic agent, and is most preferably performed by administering one or more anticancer-agents selected from the group consisting of chemotherapy agents, targeted anticancer agents, and immunotherapeutic agents, but is not limited thereto.

In another aspect, the present invention is directed to an artificial intelligence-based device for providing information for early diagnosis of cancer, the device including: a decoder configured to extract nucleic acids from a biological sample and decode sequence information; an aligner configured to align the decoded sequence with a reference genome database; a mutation detector configured to detect a mutation based on the aligned sequence information; a tumor-derived mutation detector configured to input the detected mutation into an artificial intelligence model trained to distinguish a tumor-derived mutation and determine whether or not a tumor-derived mutation is present; and a cancer diagnostic unit configured to determine that cancer or microscopic residual cancer is present when the tumor-derived mutation is detected.

In another aspect, the present invention is directed to a computer-readable storage medium including an instruction configured to be executed by a processor for providing information for early diagnosis of cancer, through the following steps including: (a) extracting nucleic acids from a biological sample to obtain sequence information; (b) aligning the sequence information (reads) with a reference genome database; (c) detecting a mutation based on the aligned sequence reads; (d) inputting the detected mutation information to an artificial intelligence model trained to distinguish tumor-derived mutations and comparing an output value with a cut-off value to determine whether or not a tumor-derived mutation is present; and (e) determining that cancer or microscopic residual cancer is present when the tumor-derived mutation is detected, wherein the artificial intelligence model in step (d) is trained to distinguish at least one feature selected from the group consisting of a functional feature of cancer, a mutation pattern, and a technical feature of mutation.

Example

Hereinafter, the present invention will be described in more detail with reference to examples. However, it will be obvious to those skilled in the art that these examples are provided only for illustration of the present invention, and should not be construed as limiting the scope of the present invention.

Example 1. Acquisition of tumor, WBC and cfDNA WGS data and determination of origin of mutations found in cfDNA

Whole genome sequencing genomic data from tumor tissue, plasma-depleted whole blood cell (WBC), and cfDNA for respective patients are required to determine whether or not a single genetic mutation found in cfDNA is a tumor-derived mutation, a haematopoiesis mutation, or an artifact. WGS samples of tumor tissue, WBC, and cfDNA of cancer patients were obtained and processed using the GATK pipeline. To secure the single gene mutation profile derived from the tumor of each patient, tumor, haematopoiesis and cfDNA mutations were detected using Mutect2.

Data used for detection are whole exome sequencing data for tumor tissue, WBC, and cfDNA of 38 metastatic breast cancer patients and are phs001417.v1.p1 data registered in the dbGaP database of Adalsteinsson, V. A. et al. Nat. Commun. 8, 1324 (2017) .

Specifically, the process of producing the obtained sequence information (reads) into bam, which is a file of a format enabling detection of mutations, was performed. The bam file is a binary format file containing information about sequence reads aligned with a reference genome database. The genome analysis tool kit (GATK) provides tools and standard analysis pipelines for NGS data analysis and the data pre-processing pipeline for mutation detection provided by GATK was used (see: https://gatk.broadinstitute.org /hc/en-us/articles/360035535912-Data-pre-processing-for-variant-discovery). The pre-processing is divided into three stages. The first step is aligning the obtained sequence information (reads) with the reference genome database. The second step is displaying duplicated sequence information (reads) generated by PCR in the process of producing sequence information (reads). The third step is base quality score recalibration of recalculating and adjusting the base quality of sequence information (reads).

Which mutation among the mutations detected in cfDNA was the single gene mutation derived from the tumor was determined using the constructed patient-specific tumor and haematopoiesis single gene mutation profiles. The result of determination with breast cancer patient samples showed that an average of 15.6% (97) of the single genetic mutations found in cfDNA was tumor-derived mutations and the artifact ratio was very high at 84%.

Example 2. Extraction of functional feature of cancer for detecting tumor-derived mutations

2-1. Transcriptome data, gene expression level and selection score extraction

Repli-seq, Dnase-seq, and ChIP-seq (H3K4me1, H3K4me3, H3K9me3, H3K27me3, H3K36me3) were obtained and pre-processed from ENCODE, and RNA-seq data of cancer patients from TCGA were used as transcriptome data. In addition, positive selection and negative selection score data for each type of cancer were also used as features of the model to be developed.

First, genome and epigenetic data of MCF7, a breast cancer cell line, were collected from ENCODE. Repliseq, Dnase-seq, and ChIP-seq (H3K4me1, H3K4me3, H3K9me3, H3K27me3, H3K36me3) of the MCF7 cell line were obtained from ENCODE. The transcriptome data used herein was transcriptome data of 1099 TCGA breast cancer patients in the Toil database. The Toil database is a large-scale transcriptome database that uniformly produces data from a large-scale transcriptome cohort through the same preprocessing process. The average of the amount of each gene expressed in breast cancer patients was calculated by calculating the average of the expression of each gene in 1099 breast cancer patients, and this average was used as a feature of the artificial intelligence model.

As breast cancer progressed, the quantitative values of genes that are more prone to or less prone to mutation depending on positive or negative selection, respectively, were used as the features of the artificial intelligence model. The quantitative value for positive selection was the average of quantitative values collected from two papers. The quantitative value for negative selection was collected from one paper.

(reference: ENCODE: https://www.encodeproject.org/)

(reference: Toil: https://doi.org/10.1038/nbt.3772)
(reference: Positive selection: 10.1016/j.cell.2017.09.042, 10.1038/ng.3987)
(reference: Negative selection: 10.1016/j.cell.2017.09.042)

2-2. Extraction of mutation signature

It has already been shown that tumor-derived mutations and haematopoiesis mutations have different molecular characteristics. Recently, it has been reported that tumor-derived single gene mutations and haematopoiesis single gene mutations have different mutational signatures (Jacom J. Chabon et al., Nature, Vol. 580, pp. 245-251, 2020). Accordingly, the characteristics of the distribution depending on the origin (tumor, haematopoiesis, artifact) of the mutations identified in the liquid biopsy were analyzed for six types (T>G, T>C, T>A, C>T, C>G, and C>A) of single gene mutations used to calculate mutational signatures using the data of Example 1. The result of the analysis showed that tumor-derived mutations, haematopoiesis mutations, and artifacts had different mutational signature patterns. Mutations identified in liquid biopsy exhibited different mutational signature patterns for each origin. Therefore, the mutational signatures were used as the features of the algorithm.

Mutational signatures were calculated using a program called "bedtools" and a python script. Bedtools is a command-line program that supports quick mutual calculation of genome data including one-dimensional coordinate systems such as BED, GFF3, and VCF. By identifying the nucleobase of the reference genome at the location of the detected mutation and the base of the detected mutation, the original base and the substituted base of the corresponding mutation were identified to determine the mutation pattern.

2-3. Extraction of single gene mutation accumulation pattern (regional mutation density, RMD)

The mechanisms by which single gene mutations occur are different for each type of cancer, and the patterns of accumulation of mutations are also different. In particular, the patterns of accumulation of passenger mutations are greatly different for each cancer type and there are previous studies that use these characteristics to classify cancer types depending on passenger mutations. Therefore, the accumulation pattern of single genetic mutations (regional mutation density) for each cancer type was used as a feature of the tumor-derived mutation detection algorithm. Haematopoiesis mutation accumulation patterns, cell-free mutation accumulation patterns in normal subjects, and germline mutation accumulation patterns in normal subjects were also used as features of the artificial intelligence model. In these examples, the breast cancer single gene mutation accumulation pattern, haematopoiesis mutation accumulation pattern, cell-free mutation accumulation pattern in normal subjects, and germline mutation accumulation pattern in normal subjects were used as features of the artificial intelligence model.

Each mutation accumulation pattern was calculated in accordance with the following method.

The whole genome was divided into sections with a certain length, the number of mutations in each section (1 Mb or 10 kb) was summed to calculate the amount of mutations in each section, and the amount of mutations in each section was divided by the total number of mutations to perform normalization.

The single gene mutation accumulation pattern for each cancer type was constructed using WGS produced by an international cancer genome project called "PCAWG" (Pan-Cancer Analysis of Whole Genomes, Campbell, P.J., Getz, G. et al., Nature 578, 82-93, 2020).

Haematopoiesis mutation accumulation patterns were constructed using blood WGS from PCAWG ovarian cancer patients.

The cell-free mutation accumulation pattern of normal subjects was constructed using cell-free WGS of 100 normal subjects from GC Genome Corporation.

The normal germline mutation accumulation patterns were constructed using the large-scale WGS of The Genome Aggregation Database (gnomAD, Karczewski, K.J. et al., Nature 581, 434-443, 2020) .

Example 3. Training of artificial intelligence algorithm to detect tumor-derived single genetic mutations

22 functional features for each cancer type obtained through the previous analysis and 26 sequencing quality features extracted from the genome data of the patient were used to develop an algorithm to detect single gene mutations derived from tumors in cfDNA. The patient genome data used herein was the genome data of Example 1. The 26 sequencing quality features were obtained by extraction at the location of the single gene mutation using a tool called "bamcount" after preprocessing liquid biopsy genome data of each patient through the gatk pipeline. The algorithm to detect single gene mutations derived from tumors was developed using a total of the 48 features extracted in this way.

The 48 extracted features are shown in Table 1 above.

The artificial intelligence algorithm is used to construct a binary classification model that distinguishes between tumor-derived mutations and the residue of single genetic mutations detected in cfDNA. Three artificial intelligence models, namely, Random Forest, XGBoost, and Deep Neural Network, were used for model training:

For optimization of the Random Forest and XGBoost models, the training data was repeatedly classified into training data and validation data through 5-fold cross validation, and hyper-parameter tuning was performed. For deep neural network optimization, hyper-parameter tuning was performed after classifying the detected mutation data into training, validation, and test data.

Experimental Example 1. Analysis of characteristics depending on origin of single genetic mutations detected in cfDNA

The characteristics of tumor-derived mutations detected in cfDNA were analyzed using the cfDNA, tumor, and WBC liquid biopsy genome data of 38 breast cancer patients of Example 1, and training and testing of the tumor-derived mutation detection algorithm were conducted.

The single gene mutations detected in the cfDNA of breast cancer patients are classified depending on the origin and mutational signatures were compared. The result of comparison showed that C>T and C>G mutations occur frequently in tumor-derived mutations, whereas C>A mutations occur frequently in artifacts, which indicates that the mutations detected in these cfDNAs had different characteristics depending on the origin thereof (FIG. 3).

In addition, the distribution of breast cancer biological features depending on the cfDNA origin of breast cancer patients was determined. It is known that there are relatively few SNVs in areas with early replication timing, and many mutations occur due to poor repair mechanisms in areas with late replication timing.

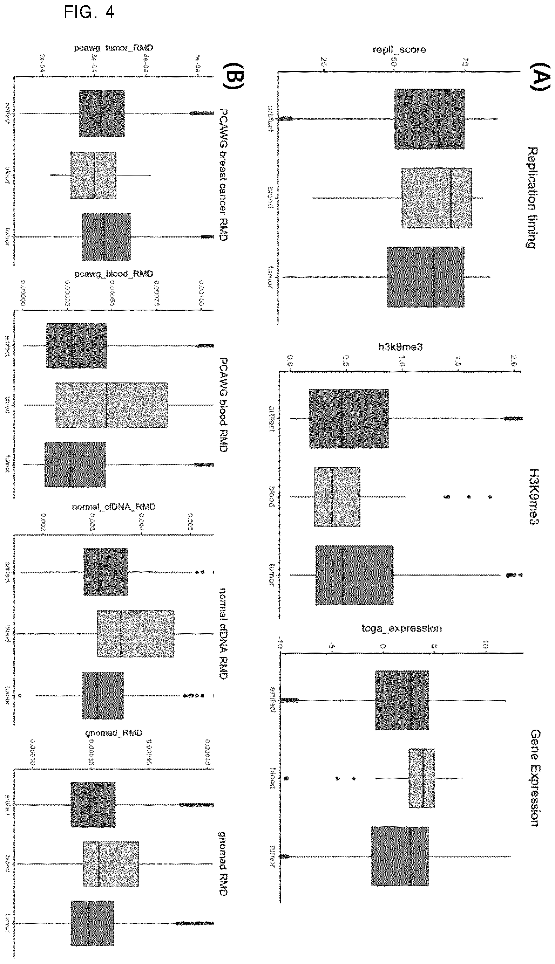

As a result, as shown in A of FIG. 4, as replication score decreases, replication timing becomes late. Consistent with the previously known mechanism, it was found that the replication score was low in the tumor mutation of cfDNA, and more tumor mutations occur in heterochromatin of breast cancer with a high H3K9me3 value, which is consistent with the previously known biological mechanism. Consistent with the feature that mutations do not occur easily in genes with high expression, the gene expression level was low in tumor mutations, which supports that biological features are important factors to distinguish tumor-derived mutations from artifacts and blood.

In addition, the result of comparison in RMD values depending on cfDNA origin of breast cancer patients, as shown in B of FIG. 4, among the biological features, the origin caused the biggest difference. That is, PCAWG breast cancer RMD tended to be higher in tumor-derived mutations than in cfDNA artifacts, and it was found that PCAWG blood, gnomAD, and normal subject cfDNA RMD were higher in cfDNA hematopoiesis mutations.

Experimental Example 2. Training and testing of artificial intelligence algorithm to detect tumor-derived single gene mutation

For training and testing of the tumor-derived single gene mutation detection algorithm, 38 patients were divided into 30 patients for training data and 8 patients for testing data. The result of testing after constructing the tumor-derived single gene mutation detection algorithm showed that the random forest and DNN showed excellent performance corresponding to ROC AUC of 0.922 and 0.864, respectively. In addition, the random forest and DNN showed excellent performance corresponding to an average precision of 0.585 (FIG. 5).

Experimental Example 3. Analysis of important feature of breast cancer tumor-derived single gene mutation detection algorithm

An analysis was conducted to determine which features among the 48 features used in algorithm training were important for detection of tumor-derived mutations.

An analysis was conducted to determine which features among the 22 functional features of cancer used in algorithm training were important for detection of tumor-derived mutations. At this time, the functional features of cancer were subdivided into 6 features related to mutational signatures and 16 biological features. The feature importance was measured using the degree to which the performance (F1 score) of the training model is deteriorated when feature values were randomly shuffled. After the process of randomly mixing and measuring the performance of the model was performed a total of 100 times, the average degree of model performance deterioration was measured.

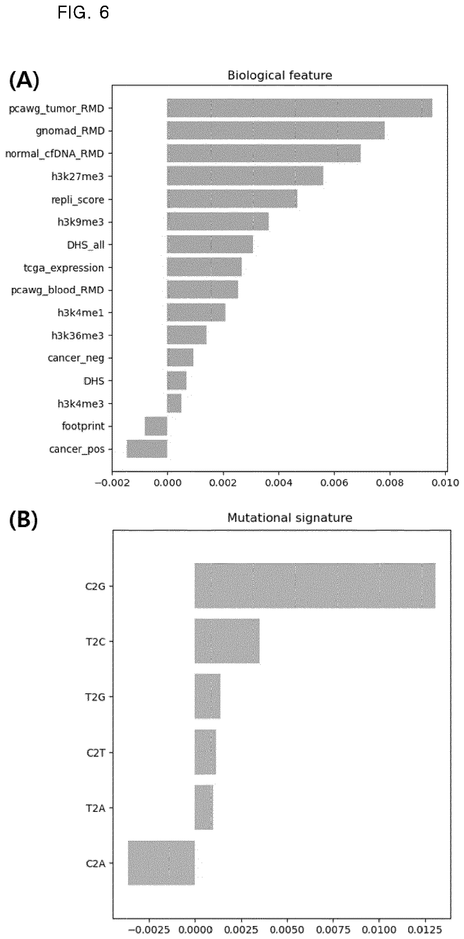

The result showed that the mutation accumulation pattern (regional mutation density) plays the most important role in detecting tumor-derived mutations, as shown in FIG. 6. Three mutation accumulation pattern features are ranked in biological feature importance 1, 2, and 3, respectively, and thereamong, the breast cancer mutation accumulation pattern (pcawg_tumor_rmd) plays the most important role. In addition, it was found that the histone modification marker, H3K27me3, and DNA replication timing played an important role.

Experimental Example 4. Prediction of mutational signature using developed algorithm

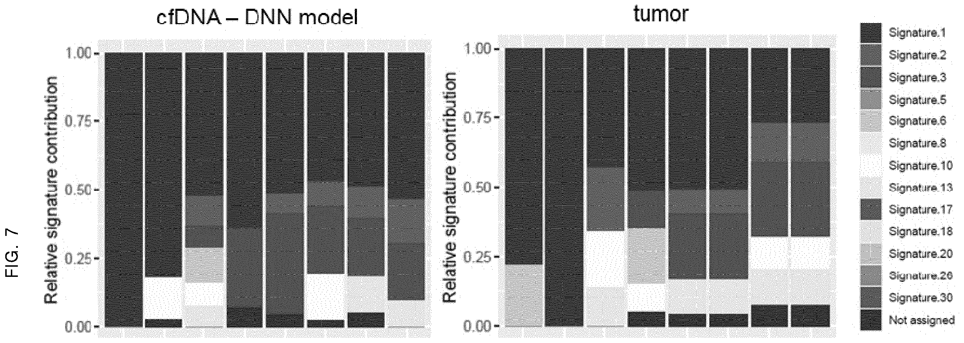

Whether or not the algorithm developed in this study could actually predict cancer mutational signature patterns was verified. The results of analysis of the mutational signature using the tumor-derived mutation predicted through the algorithm developed in this study was compared with the result of analysis of the mutational signature in tumors predicted using the algorithm developed in this study (FIG. 7).

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

[Industrial applicability]

The method for detecting tumor-derived mutations in cell-free DNA and the early diagnosis for cancer using the method according to the present invention are highly industrially applicable and are thus useful for early cancer diagnosis because they provide early diagnosis for cancer with high accuracy and sensitivity using both functional and sequence features of cancer based on artificial intelligence through next generation sequencing (NGS) .

**Claims**

1. An artificial intelligence-based method for detecting a tumor-derived mutation in cell-free DNA, the method comprising:

   (a) extracting nucleic acids from a biological sample to obtain sequence information;
   (b) aligning the sequence information (reads) with a reference genome database;
   (c) detecting a mutation based on the aligned sequence reads; and
   (d) inputting the detected mutation information to an artificial intelligence model trained to distinguish a tumor-derived mutation and comparing an output value with a cut-off value to determine whether or not a tumor-derived mutation is present,

   wherein the artificial intelligence model in step (d) is trained to distinguish the tumor-derived mutation based on at least one feature selected from the group consisting of a functional feature of cancer, a mutation pattern, and a technical feature of mutation.

2. The artificial intelligence-based method according to claim 1, wherein step (a) comprises:

   (a-i) obtaining nucleic acids from a biological sample;
   (a-ii) removing proteins, fats, and other residues from the obtained nucleic acids using a salting-out method, a column chromatography method, or a bead method to obtain purified nucleic acids;
   (a-iii) producing a single-end sequencing or paired-end sequencing library for the purified nucleic acids or nucleic acids randomly fragmented by enzymatic digestion, pulverization, or hydroshearing;
   (a-iv) reacting the produced library with a next-generation sequencer; and
   (a-v) obtaining sequence information (reads) of the nucleic acids in the next-generation sequencer.

3. The artificial intelligence-based method according to claim 1, further comprising:
   selecting reads having a mapping quality score of the aligned nucleic acid fragments equal to or greater than a cut-off value prior to step (c).

4. The artificial intelligence-based method according to claim 3, wherein the cut-off value is 50 to 70.

5. The artificial intelligence-based method according to claim 1, wherein the step (c) of detecting the mutation comprises:

   (c-i) selecting a nucleotide sequence different from the reference genome in the aligned reads; and
   (c-ii) storing the selected nucleotide sequence information.

6. The artificial intelligence-based method according to claim 1, wherein the functional feature of cancer in step (d) comprises at least one feature selected from the group consisting of (i) a single genetic mutation accumulation patterns (regional mutation density, RMD), and (ii) replication timing, H3K4Me1, H3K4Me3, H3K9Me3, H3K27Me3, H3K36Me3, Dnase I hypersensitive site (DHS), an amount of protein binding site (footprint) gene expression in DHS, a cancer positive selection score and a cancer negative selection score.

7. The artificial intelligence-based method according to claim 1, wherein the mutation pattern in step (d) comprises at least one selected from the group consisting of C -> A, C -> G, C -> T, T -> A, T -> C, and T -> G.

8. The artificial intelligence-based method according to claim 1, wherein the technical feature of mutation in step (d) comprises at least one selected from the group consisting of:

an average read depth, an average mapping quality, an average base quality, an average number of mismatches, an average of reference allele positions, an average of base quality sums of mismatches, the number or position of bases having a Phred quality of 2 at a 3' end, an average clipped read length, an average of positions from a read 3' end, a ratio of plus strand reads, and a DNA fragment length of a reference allele of the mutation region;

an average read depth, an average mapping quality, an average base quality, an average number of mismatches, an average of reference allele positions, an average of base quality sums of mismatches, the number or position of bases having a Phred quality of 2 at a 3' end, an average clipped read length, an average of positions from a read 3' end, a ratio of plus strand reads, a DNA fragment length, and a DNA fragment ratio of a variant allele of the mutation region; and

MUT.notBoth (defined as the number of DNA fragments that do not overlap at mutation positions in forward and reverse reads + the number of DNA fragments that overlap at mutation positions in forward and reverse reads, but have different mutations).

9. The artificial intelligence-based method according to claim 1, wherein the technical feature of step (d) comprises features shown in Table 1.

10. The artificial intelligence-based method according to claim 1, wherein the artificial intelligence model in step (d) is trained to determine whether a tumor-derived mutation is correct or not.

11. The artificial intelligence-based method according to claim 10, wherein the artificial intelligence model comprises at least one selected from the group consisting of random forest, XGboost, and deep neural network.

12. A method for providing information for early diagnosis of cancer, the method comprising:

(a) detecting a tumor-derived mutation in cell-free DNA by the method according to any one of claims 1 to 11; and
(b) determining that cancer or microscopic residual cancer is present when the tumor-derived mutation is detected.

13. An artificial intelligence-based device for providing information for early diagnosis of cancer, the device comprising:

a decoder configured to extract nucleic acids from a biological sample and decode sequence information;
an aligner configured to align the decoded sequence with a reference genome database;
a mutation detector configured to detect a mutation based on the aligned sequence information;
a tumor-derived mutation detector configured to input the detected mutation into an artificial intelligence model trained to distinguish a tumor-derived mutation and determine whether or not a tumor-derived mutation is present; and
a cancer diagnostic unit configured to determine that cancer or microscopic residual cancer is present when the tumor-derived mutation is detected.

14. A computer-readable storage medium including an instruction configured to be executed by a processor for providing information for early diagnosis of cancer, through the following steps comprising:

(a) extracting nucleic acids from a biological sample to obtain sequence information;
(b) aligning the sequence information (reads) with a reference genome database;
(c) detecting a mutation based on the aligned sequence reads;
(d) inputting the detected mutation information to an artificial intelligence model trained to distinguish tumor-derived mutations and comparing an output value with a cut-off value to determine whether or not a tumor-derived mutation is present; and
(e) determining that cancer or microscopic residual cancer is present when the tumor-derived mutation is detected,

wherein the artificial intelligence model in step (d) is trained to distinguish the tumor-derived mutation based on at least one feature selected from the group consisting of a functional feature of cancer, a mutation pattern, and a technical feature of mutation.

15. A method for early diagnosis of cancer, the method comprising:

(a) detecting a tumor-derived mutation in cell-free DNA by the method according to any one of claims 1 to 11; and
(b) determining that cancer or microscopic residual cancer is present when the tumor-derived mutation is detected.

FIG. 1

FIG. 2

FIG. 3

Nature **578**, 94–101 (2020)

FIG. 4

FIG. 5

(A) ROC Curve Analysis

(B) Precision Recull Curve

FIG. 6

**(A)**

**(B)**

FIG. 7

| INTERNATIONAL SEARCH REPORT | International application No. |
|---|---|
| | **PCT/KR2022/004189** |

**A. CLASSIFICATION OF SUBJECT MATTER**

**G16H 50/20**(2018.01)i; **G16H 50/50**(2018.01)i; **G16H 50/70**(2018.01)i; **G16B 30/10**(2019.01)i; **G16B 20/20**(2019.01)i; **C12Q 1/6869**(2018.01)i; **C12Q 1/6806**(2018.01)i; **G06N 20/00**(2019.01)i; **G06N 3/08**(2006.01)i

According to International Patent Classification (IPC) or to both national classification and IPC

**B. FIELDS SEARCHED**

Minimum documentation searched (classification system followed by classification symbols)

G16H 50/20(2018.01); G16B 30/00(2019.01); G16B 40/00(2019.01); G16B 50/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인공지능 (Artificial Intelligence), 무세포 DNA (cell-free DNA, cfDNA), 종양 (cancer), 변이 (mutation), 검출 (detection), 암진단 (cancer diagnosis)

**C. DOCUMENTS CONSIDERED TO BE RELEVANT**

| Category* | Citation of document, with indication, where appropriate, of the relevant passages | Relevant to claim No. |
|---|---|---|
| A | KR 10-2019-0085667 A (GREEN CROSS GENOME CORPORATION) 19 July 2019 (2019-07-19)<br>See abstract; paragraph [0020]; and claims 1-15. | 1-15 |
| A | KR 10-2019-0053695 A (UNIVERSITY OF ULSAN FOUNDATION FOR INDUSTRY COOPERATION et al.) 20 May 2019 (2019-05-20)<br>See entire document. | 1-15 |
| A | WANG, J. et al. CNNdel: calling structural variations on low coverage data based on convolutional neural networks. BioMed Research International. 2017, thesis no. 6375059, pp. 1-9.<br>See entire document. | 1-15 |
| A | CARIO, C. L. et al. A machine learning approach to optimizing cell-free DNA sequencing panels: with an application to prostate cancer. BMC cancer. 2020, vol. 20, thesis no. 820, pp. 1-9.<br>See entire document. | 1-15 |
| A | WAN, N. et al. Machine learning enables detection of early-stage colorectal cancer by whole-genome sequencing of plasma cell-free DNA. BMC cancer. 2019, vol. 19, thesis no. 832, pp. 1-10.<br>See entire document. | 1-15 |

☐ Further documents are listed in the continuation of Box C.　　☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "D" document cited by the applicant in the international application | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **04 July 2022** | **05 July 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

INTERNATIONAL SEARCH REPORT
Information on patent family members

International application No.

**PCT/KR2022/004189**

| Patent document cited in search report | | | Publication date (day/month/year) | Patent family member(s) | | | Publication date (day/month/year) |
|---|---|---|---|---|---|---|---|
| KR | 10-2019-0085667 | A | 19 July 2019 | KR | 10-2029393 | B1 | 07 October 2019 |
| | | | | WO | 2019-139363 | A1 | 18 July 2019 |
| KR | 10-2019-0053695 | A | 20 May 2019 | CN | 110070915 | A | 30 July 2019 |
| | | | | KR | 10-2071491 | B1 | 30 January 2020 |
| | | | | SG | 11202004159 | A | 29 June 2020 |
| | | | | WO | 2019-093814 | A2 | 16 May 2019 |
| | | | | WO | 2019-093814 | A3 | 11 July 2019 |

Form PCT/ISA/210 (patent family annex) (July 2019)

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JACOB J. CHABON et al.** *Nature,* 2020, vol. 580, 245-25
- **METZKER, M.** *Nature Biotechnology Reviews,* 2010, vol. 11, 31-46
- **ADALSTEINSSON, V. A. et al.** *Nat. Commun.,* 2017, vol. 8, 1324
- **JACOM J. CHABON et al.** *Nature,* 2020, vol. 580, 245-251
- **CAMPBELL, P.J. ; GETZ, G. et al.** Pan-Cancer Analysis of Whole Genomes. *Nature,* 2020, vol. 578, 82-93
- **KARCZEWSKI, K.J. et al.** *Nature,* 2020, vol. 581, 434-443